# EUROPÄISCHE PATENTANMELDUNG

(11) **EP 1 839 666 A1**
(43) Veröffentlichungstag der Anmeldung: **03.10.2007**
(21) Anmeldenummer: 06090025.5
(22) Anmeldetag: 15.02.2006
(51) Int. Cl.: A61K 33/06, A61K 36/16, A61K 36/8962, A61K 36/26, A61K 36/734, A61K 36/185, A61P 9/12

(54) **Bluthochdruck-Mittel enthaltend tribomechanisch aktivierte Zeolithen**

(71) Anmelder: Lelas, Tihomir, 10040 Zagreb (HR); Ljubicic, Mijo, 10585 Berlin (DE); Ivkovic, Slavko, 10000 Zagreb (HR)
(72) Erfinder: Lelas, Tihomir, 10040 Zagreb (HR); Ljubicic, Mijo, 10585 Berlin (DE); Ivkovic, Slavko, 10000 Zagreb (HR)
(74) Vertreter: Boeckh, Tobias

(57) **Zusammenfassung**

Die Erfindung betrifft pharmazeutische Mittel umfassend 50 - 90 Gew.-% TMAZ, 1 - 30 Gew.-% Bärlauchkraut, 1 - 10 Gew.-% Mistelkraut, 1 - 10 Gew.-% Silberlindenblüten, 1 - 10 Gew.-% Weißdornblüten und/oder 1 - 10 Gew.-% Gingkoblätter sowie die Verwendung des pharmazeutischen Mittels zur Behandlung von Krankheiten, die mit Bluthochdruck assoziiert sind.

## Beschreibung

Die Erfindung betrifft pharmazeutische Mittel umfassend 50 - 90 Gew.-% TMAZ, 1 - 30 Gew.-% Bärlauchkraut, 1 - 10 Gew.-% Mistelkraut, 1 - 10 Gew.-% Silberlindenblüten, 1 - 10 Gew.-% Weißdornblüten und/oder 1 - 10 Gew.-% Gingkoblätter sowie die Verwendung des pharmazeutischen Mittels zur Behandlung von Krankheiten, die mit Bluthochdruck assoziiert sind.

Den erhöhten Druck in den Arterien, der eine längere Zeit anhält, nennt man hohen Blutdruck oder Hypertension, die infolge des Versagens des Blutdruckregelungssystems auftritt.
Blutdruckwerte von 140 bis 159 (obere) und 90 bis 99 (untere) stellen eine Hypertension ersten Grades dar, während bei Werten ab 160 (obere) und 100 (untere) eine Hypertension zweiten Grades vorliegt.
Jede vierte Person leidet an Bluthochdruck, mit steigender Tendenz, da die Zahl der Erkrankten, insbesondere im Zusammenhang mit dem Stoffwechselsyndrom, jedes Jahr steigt.

In den letzten 40 Jahren ist die Behandlung des Bluthochdrucks durch die Entdeckung neuer Medikamente immer effektiver geworden, sodass durch deren Kombination das Stadium der malignen Hypertension verschwindet, bei dem ein hohes Todesrisiko bestand, und die Hypertension zu einer heilbaren Krankheit geworden ist, vorausgesetzt die Medikamente werden eingenommen. Bei längerer Einnahme (sie werden ein Leben lang eingenommen) können sie jedoch hepatotoxisch und nephrotoxisch sein und stark ausgeprägte Nebenwirkungen hervorrufen. Ein besonderes Problem ist die Resistenz. Daher ist es erforderlich, die Antihypertensiva häufig zu wechseln, sonst tritt eine Indikation der Unwirksamkeit der Anwendung der Kombitherapie auf.

**Nebenwirkungen, die von bekannten Antihypertensiva hervorgerufen werden:**

### 1. Betablocker

a) Vasokonstriktion der peripheren Blutadern (kalte Füße)
b) Impotenz

### 2. ACE-Inhibitoren (Angiotenzin-Converting-Enzym)

a) Verringerung der glomerulären Filtration
b) Wasserretention
c) Proteinurie
d) Veränderungen an den glomerulären (Nieren-) Blutadern

### 3. Alphablocker

a) Orthostatische Hypotensionen
b) Reboundeffekt
c) Effekt der ersten Dosis

### 4. Zentrale adrenergische Inhibitoren

a) Orthostatische Hypotensionen
b) Depression

### 5. Diuretika

a) Kontrainsulärer Effekt
b) Hyponatrium
c) Hypokalium
d) Verringerung der Pärfusion durch die periphere Zirkula tion

### 6. Calciumkanal-Blocker

a) in Kombination mit Betablocker, Gefahr von Herzarrest

Ein besonders großes Problem bei der konventionellen Behandlung mit oben angeführten Medikamenten und verschiedenen Kombinationen ist, dass der systolische (obere) und diastolische (untere) Blutdruck nicht divergent (gleichmäßig) fallen.
Der systolische Blutdruck fällt auf das gewünschte Niveau, aber der diastolische Blutdruck bleibt erhöht, was den Unterschied zwischen den beiden Werten verringert und die periphere Zirkulation, d. h. den Blutdurchfluss durch die Organe, gefährdet.

Mit der erfolgreichen Behandlung der Hypertension hat sich die aus Komplikationen bei Herzkrankheiten resultierende Sterblichkeit um 56% verringert und die Sterblichkeit infolge von Hirnschlag sogar um 70%.
Ein großes Problem besteht jedoch darin, dass 30% der Patienten nicht wissen, dass sie einen hohen Blutdruck haben und 11% von ihnen ihn trotz Diagnose nicht oder nicht adäquat behandeln. Nur 34% der Bluthochdruckpatienten haben ihre Krankheit unter Kontrolle.
Immerhin 5 - 10% der Hypertensionen sind widerstandsfähig auf Therapie, trotz der Kombination von 3 Antihypertensiva, einschließlich Diuretikum. Die Blutdruckwerte fallen nicht unter 140/90.

Die Bluthochdruckkrankheit stellt heute einen großen Risikofaktor in der Entstehung dauerhafter Schäden großer und kleiner Blutadern in Niere, Herz, Hirn und peripherer Zirkulation dar, was letztlich Hirn- und Herzschlag sowie Arterienstenose verursacht.
In den letzte 20 Jahren ist die Hypertension keine tödliche Krankheit mehr und das Stadium der malignen Hypertension ist dank der kontinuierlichen und wirksamen Therapie immer seltener.

Der Arteriendruck in den großen Blutadern ist das Verhältnis von Volumen und Durchmesser der Blutadern, d. h. der Vasokonstriktion oder der Dilatation der Blutadern.
Der maximale Blutdruck (der systolische - während der Herzsystole bzw. des Blutauswurfs aus der linken Klappe in die Aorta) hängt von der Elastizität der Blutadern ab, die durch die aortosklerotischen Ablagerungen in der Wand der großen Arterien sinkt, die den systolischen Blutschlag kompensieren. Der minimale Blutdruck in den Arterien oder der diastolische (während des Anpumpens der rechten Herzklappe) hängt ausschließlich von der peripheren Resistenz in den kleine Blutadern ab.
Der Durchfluss durch die kleinen und großen Blutadern hängt von den Blutdruckregulatoren ab, damit ein ausreichender Durchfluss durch alle Organe aufrechterhalten wird.

Aufgabe der Erfindung war es daher, die Nachteile des Standes der Technik zu beseitigen und ein einfaches, sicheres und effektive Mittel zur Behandlung von Krankheiten, die mit Bluthochdruck assoziiert sind, bereitzustellen.

Die Erfindung löst dieses Problem durch ein pharmazeutisches Mittel umfassend 50 - 90 Gew.-% TMAZ, 1 - 30 Gew.-% Bärlauchkraut, 1 - 10 Gew.-% Mistelkraut, 1 - 10 Gew.-% Silberlindenblüten, 1 - 10 Gew.-% Weißdornblüten und/oder 1 - 10 Gew.-% Gingkoblätter. Weitere vorteilhafte Ausgestaltungsformen der Erfindung ergeben sich durch die Neben- und Unteransprüche.

Es war völlig überraschend, dass mit der erfindungsgemäßen Kombination Bluthochdruck behandelt werden kann, wobei die Kombination der einzelnen Bestandteile des pharmazeutischen Mittels zu einem synergetischen Effekt führt, der deutlich über einen additiven Effekt der einzelnen kombinierten Bestandteile hinausgeht. Es war im Stand der Technik nicht bekannt, gerade die erfindungsgemäßen Elemente in der erfindungsgemäßen Kombination zusammenwirken zu lassen. Durch die Kombination wird ein unerwarteter Gesamterfolg erzielt, der insbesondere in einem synergetischen Effekt mündet, der dazu führt, dass die wichtigsten Blutdruckregulatoren so beeinflusst werden, dass eine Normalisierung des Blutdruckes eintritt. Erfindungsgemäß werden unter den wichtigsten Blutdruckregulatoren die volumenmäßige und die vasokonstriktorische (Verengung der Blutadern) verstanden. Die volumenmäßige Regulation beim Herzen erfasst erfindungsgemäß die Kraft, mit der das Herz das Blut in die Aorta transportiert, sowie die Geschwindigkeit des Blutkreislaufes. Die Arterien haben die Möglichkeit, sich zu erweitern und sich zusammenzuziehen und regeln so den Blutdruck. Bei größerer Elastizität der Arterien ist der Widerstand selbstverständlich kleiner und der Blutdruck wird dann erfolgreicher geregelt bzw. bewegt sich im normalen Bereich.
Bei den Nieren: Sie regeln die Natriummenge im Blut. Das Natrium regelt das Volumen im zirkulierenden Raum; sowie bei den Hormonen: Aldosteron, Esatrogene, Kortikosteroide.

Zu der vasokonstriktorischen Blutdruckregulierung zählt im Sinne der Erfindung die Regulierung über die Niere: Mesangiale Zellen im Glomerulum kontrollieren die Aktivität des zirkulierenden Ang II, wodurch der Blutdruck im ganzen Arteriensystem geregelt wird. Lokal wird auch der Durchfluss durch die Nieren geregelt, der zweierlei Möglichkeiten hat, sowohl das Volumen zu kontrollieren als auch Widerstand in den Arterien zu leisten; vaskulare Krankheiten der Nierenarterien; beim Hirn: Sondert ebenfalls Ang II ab, um den eigenen Durchfluss aufrechtzuerhalten. Es ist bekannt, dass jede Störung beim Durchfluss durch die Hirnarterien einen systematischen Anstieg des Arteriendrucks hervorruft; Plazenta: Hat ebenfalls die Möglichkeit der Blutdruckregulierung durch Absonderung von Ang II. Es ist bekannt, dass es während der Schwangerschaft, wenn die plazentare Zirkulation gefährdet ist, bei der Schwangeren zur systematischen Hypertension kommt; Lunge: Hat eine eigene Kontrolle des Arteriendrucks Renin-Angiotensin ACE Ang II-System; Barorezeptoren: Sind knotige Strukturen, die sich in der Wand der Blutadern befinden, die bei der Druckänderung ein Signal ans Hirn senden, die Herzfunktion entweder zu verlangsamen oder zu beschleunigen bzw. die Arterien entweder zu erweitern oder zu verengen, damit der Druck im normalen Bereich bleibt. Auch Adrenalin und Noradrenalin sind starke Vasokonstriktoren. Auch das Endotel der Blutadern führt zu einer lokalen Regulierung der Vasokonstriktion oder der Dilatation; weiterhin wirkt Eiweiß-Endotelin als starker Vasokonstriktor. Auch Radikale wie z. B. NO, sind ein ausschlaggebender Faktor in Bezug auf die Erweiterung der Blutadern. Die bekannten Wirkungsmechanismen der antihypertensiven Therapie sind:
- zentrale adrenergische Inhibitoren
- Ang II Blocker
- Blocker des Converting-Enzyms ACE
- Blocker des Calciumkanals
- Betablocker
- Alphablocker
- Periphere Dilatatoren
- Diuretika

Gemäß dieser bekannten Wirkmechanismen wird angestrebt, dass ein gutes Antihypertensivum wie folgt wirkt:

Eine Wirkung auf das Volumen das zirkulierenden Raums:
- Diuretikumeffekt
- Adosteroneffekt
- die K- und Na-Konzentration im zirkulierenden Raum sowie in der Wand der Blutadern sowie die Vasokonstriktion der Blutadern (der großen und der kleinen):
   - Empfindlichkeit des Rezeptors auf Vasokonstriktoren insbesondere auf Alpha und Beta
   - c-AMP, c-GMP - postrezeptorische Messenger -
   - Einfluss auf K/Na-Pumpen
   - Ca-Kanal
   - Aktivität Ang I - Angiotension Converting Enzym - Angiotensin II
   - Aktivität des Ang II-Rezeptors, ACE Inhibition
   - Noradrenalin
   - Blocker des Calciumkanals
   - Zentrale adrenergische Inhibition
   - Dilatatoren
   - Regulierung der Aktivität des zirkulierenden Ang II in den glomerulären mesangialen Zellen, die von der Na-Anwesenheit in der Zirkulation abhängt.

Das erfindungsgemäße Produkt Nanopressan enthält natürliche Substanzen, die mit TMAZ in der Kollisionsmaschine für die tribomechanische Aktivierung (TMA) die biophysikalische Wirkung der natürlichen Produkte steigert. In Verbindung mit TMA-Zeolithen schaffen die natürlichen Produkte neue Moleküle mit stärkeren und ganz neuen Wirkungen.

Nach Anwendung der Technologie ändert sich:
- die Partikelgröße
- Oberfläche
- elektrostatische Ladung
- Ionenaustauschkapazität
- Struktur
- Intensivere biologischen Wirkung

Das erfindungsgemäße Mittel umfasst:
1. Bärlauch (Illium ursinum) Wirkt hauptsächlich auf die Elastizität der Blutadern. Verhindert die Entstehung der arteriosklerotischen Plaques bzw. stimuliert die Funktion der Arterienglattmuskeln. Eine gute Oxidation schützt auf jeden Fall bei LDL. Wirkt auch als ACE-Inhibitor, d. h. es verhindert die Entstehung von ANG II (zeigt den präventiven Effekt, ebenso die Wirksamkeit bei eingetretener Hypertension).
2. Mistel (Viscum album) Die Erfahrungen zeigen, dass Viscotoxine eine antihypertensiven Effekt aufweisen, indem sie auf die Vasokonstriktion der glatten Muskulatur in den Blutadern wirken.
3. Silberlindenblüte (Tiliaae flos) Enthält Flavonoide, Flavone
4. Weißdornblüten (Crataegus monogyna) Enthält Flavone, Flavonoide, Procyanide; wirkt auf K/Na-ATP-asen; wirkt auch als ACE-Inhibitor sowie auf die Herzfrequenz.
5. Gingkoblätter Antioxidative Wirkung; Polyphenole, Flavonoide verstärken die Pärfusionen durch die kleinen Blutadern und verstärken ihre Elastizität.
6. TMAZ - Alumosilikate - Kieselsäure Hat eine antioxidative und diuretische Wirkung.

Die anmeldungsgemäße Lehre zeichnet sich durch folgende Merkmale aus.
- Abkehr von technisch üblichen
- neue Aufgabenstellung
- Vorliegen eines seit langem ungelösten dringenden Bedürfnisses für die Lösung des mit der Erfindung gelösten Problems
- bisheriges vergebliches Bemühen der Fachwelt
- Einfachheit einer Lösung spricht für erfinderische Tätigkeit, insbesondere wenn sie kompliziertere Lehren ersetzt
- Entwicklung der wissenschaftlichen Technik ging in eine andere Richtung
- entwicklungsstraffende Leistung
- Fehlvorstellungen der Fachwelt über die Lösung des entsprechenden Problems (Vorurteil)
- technischer Fortschritt, wie z. B.: Verbesserung, Leistungssteigerung, Verbilligung, Ersparnis an Zeit, Material, Arbeitsstufen, Kosten oder schwer beschaffbaren Rohstoffen, erhöhte Zuverlässigkeit, Beseitigung von Fehlern, Qualitätshebung, Wartungsfreiheit, größere Effektivität, höhere Ausbeute, Vermehrung der technischen Möglichkeiten, Bereitstellung eines weiteren (nicht notwendig besseren) Mittels, Eröffnung eines zweiten (nicht notwendigerweise besseren) Weges, Eröffnung eines neuen Gebietes, erstmalige Lösung einer Aufgabe, Reservemittel, Alternativen, Möglichkeit der Rationalisierung, Automatisierung oder Miniaturisierung oder Bereichung des Arzneimittelschatzes
- glücklicher Griff (wird aus einer Vielzahl von Möglichkeiten eine bestimmte gewählt, deren Ergebnis nicht vorausgesagt werden konnte, so handelt es sich um ein patentwürdigen glücklichen Griff)
- Irrtum in Entgegenhaltungen
- junges Gebiet der Technik
- Kombinationserfindung, d.h. mehrere bekannte Elemente werden zu einer Kombination zusammengeführt, die einen überraschenden Effekt aufweist
- Lizenzvergabe
- Lob der Fachwelt und
- wirtschaftlicher Erfolg.

Es war überraschend, dass die anmeldungsgemäßen Verbindungen und Kombinationsmittel prophylaktisch und therapeutisch zur Herstellung eines Mittels zur Behandlung von Bluthochdruck sowie den mit Bluthochdruck assoziierten Erkrankungen verwendet werden können. In diesem Falle werden die Kombinationsmittel und die erfindungsgemäßen Mittel als pharmazeutische Mittel eingesetzt.

Selbstverständlich ist es möglich, dass die erfindungsgemäßen Verbindungen übliche Hilfsstoffe, bevorzugt Träger, Adjuvantien und/oder Vehikel umfassen. Bei den Trägern kann es sich beispielsweise um Füllmittel, Streckmittel, Bindemittel, Feuchthaltemittel, Sprengmittel, Lösungsverzögerer, Resorptionsbeschleuniger, Netzmittel, Adsorptionsmittel und/oder Gleitmittel handeln. In diesem Fall werden die Verbindungen insbesondere als Arzneimittel oder pharmazeutisches Mittel bezeichnet.

In einer weiteren bevorzugten Ausführungsform der Erfindung wird das erfindungsgemäße Mittel als Gel, Puder, Pulver, Tablette, Retard-Tablette, Premix, Emulsion, Aufgussformulierung, Tropfen, Konzentrat, Granulat, Sirup, Pellet, Boli, Kapsel, Aerosol, Spray und/oder Inhalat zubereitet und/oder in dieser Form angewendet. Die Tabletten, Dragees, Kapseln, Pillen und Granulate können mit den üblichen, gegebenenfalls Opakisierungsmitteln enthaltenden, Überzügen und Hüllen versehen sein und auch so zusammengesetzt sein, dass sie den oder die Wirkstoffe nur oder bevorzugt in einem bestimmten Teil des Intestinaltraktes gegebenenfalls verzögert abgeben, wobei als Einbettungsmassen zum Beispiel Polymersubstanzen und Wachse verwendet werden können.

Die Arzneimittel dieser Erfindung können beispielsweise zur oralen Verabreichung in einer beliebigen oral verträglichen Dosierungsform verwendet werden, die Kapseln, Tabletten und wässrige Suspensionen und Lösungen einschließt, aber nicht darauf beschränkt ist. Im Fall von Tabletten zur oralen Verwendung schließen Träger, die häufig verwendet werden, Lactose und Maisstärke ein. Gleitmittel, wie Magnesium-stearat, können typischerweise zugesetzt werden. Zur oralen Verabreichung in Kapselform werden verwendbare Verdünnungsmittel wie Lactose und getrocknete Maisstärke eingesetzt. Wenn wässrige Suspensionen oral verabreicht werden, wird der Wirkstoff mit Emulgier- und Suspendiermitteln kombiniert. Falls gewünscht, können bestimmte Süßmittel und/oder Geschmacksstoffe und/oder Farbmittel zugesetzt werden.

Der oder die Wirkstoffe können gegebenenfalls mit einem oder mehreren der oben angegebenen Trägerstoffe auch in mikroverkapselter Form vorliegen.

Suppositorien können neben dem oder den Wirkstoffen die üblichen wasserlöslichen oder wasserunlöslichen Trägerstoffe enthalten, zum Beispiel Polyethylenglycole, Fette, zum Beispiel Kakaofett und höhere Ester (zum Beispiel C₁₄-Alkohol mit C₁₆-Fettsäure) oder Gemische dieser Stoffe.

Salben, Pasten, Cremes und Gele können neben dem oder den Wirkstoffen die üblichen Trägerstoffe enthalten, zum Beispiel tierische und pflanzliche Fette, Wachse, Paraffine, Stärke, Tragant, Cellulosederivate, Polyethylenglycole, Silikone, Bentonite, Kieselsäure, Talkum und Zinkoxid oder Gemische dieser Stoffe.

Puder und Sprays können neben dem oder den Wirkstoffen die üblichen Trägerstoffe enthalten, zum Beispiel Milchzucker, Talkum, Kieselsäure, Aluminiumhydroxid, Calciumsilikat und Polyamidpulver oder Gemische dieser Stoffe. Sprays können zusätzlich die üblichen Treibmittel, zum Beispiel Chlorfluorkohlenwasserstoffe, enthalten.

Lösungen und Emulsionen können neben den Wirkstoffen, das heißt der erfindungsgemäßen Verbindungen, die üblichen Trägerstoffe wie Lösungsmittel, Lösungsvermittler und Emulgatoren, zum Beispiel Wasser, Ethylalkohol, Isopropylalkohol, Ethylcarbonat, Ethylacetat, Benzylalkohol, Benzylbenzoat, Propylenglykol, 1,3-Butylenglykol, Dimethylformamid, Öle, insbesondere Baumwollsaatöl, Erdnussöl, Maiskeimöl, Olivenöl, Rizinusöl und Sesamöl, Glycerin, Glycerinformal, Tetrahydofurfurylalkohol, Polyethylenglycole und Fettsäureester des Sorbitans oder Gemische dieser Stoffe enthalten. Zur parenteralen Applikation können die Lösungen und Emulsionen auch in steriler und blutisotonischer Form vorliegen.

Suspensionen können neben den Wirkstoffen die üblichen Trägerstoffe wie flüssige Verdünnungsmittel, zum Beispiel Wasser, Ethylalkohol, Propylenglykol, Suspendiermittel, zum Beispiel ethoxilierte Isostearylalkohole, Polyoxyethylen-sorbit- und Sorbitan-Ester, mikrokristalline Cellulose, Aluminiummetahydroxid, Bentonit, Agar-Agar und Tragant oder Gemische dieser Stoffe enthalten.

Die Arzneimittel können in Form einer lyophilisierten sterilen injizierbaren Zubereitung, zum Beispiel als sterile injizierbare wässrige oder ölige Suspension, vorliegen. Diese Suspension kann auch mit im Fachgebiet bekannten Verfahren unter Verwendung geeigneter Dispergier- oder Netzmittel (wie zum Beispiel Tween 80) und Suspendiermittel formuliert werden. Die sterile injizierbare Zubereitung kann auch eine sterile injizierbare Lösung oder Suspension in einem ungiftigen parenteral verträglichen Verdünnungs- oder Lösungsmittel, zum Beispiel eine Lösung in 1,3-Butandiol, sein. Zu den verträglichen Vehikeln und Lösungsmitteln, die verwendet werden können, gehören Mannit, Wasser, Ringer-Lösung und isotonische Natriumchloridlösung. Außerdem werden üblicherweise sterile, nichtflüchtige Öle als Lösungsmittel oder Suspendiermedium verwendet. Zu diesem Zweck kann ein beliebiges mildes nichtflüchtiges Öl einschließlich synthetischer Mono- oder Diglyceride verwendet werden. Fettsäuren, wie Ölsäure und ihre Glyceridderivate sind bei der Herstellung von Injektionsmitteln verwendbar, wie es natürliche pharmazeutisch verträgliche Öle, wie Olivenöl oder Rizinusöl, insbesondere in ihren polyoxyethylierten Formen sind. Diese Öllösungen oder Suspensionen können auch einen langkettigen Alkohol oder einen ähnlichen Alkohol als Verdünnungs- oder Dispergiermittel enthalten.

Die genannten Formulierungsformen können auch Färbemittel, Konservierungsstoffe sowie geruchs- und geschmacksverbesserte Zusätze, zum Beispiel Pfefferminzöl und Eukalyptusöl und Süßmittel, zum Beispiel Saccharin, enthalten. Die erfindungsgemäßen Verbindungen sollen in den aufgeführten pharmazeutischen Zubereitungen vorzugsweise in einer Konzentration von etwa 0,01 bis 99,9, vorzugsweise von etwa 0,05 bis 99 Gew.-% der Gesamtmischung vorhanden sein.

Die aufgeführten pharmazeutischen Zubereitungen können außer den Verbindungen, weitere pharmazeutische Wirkstoffe enthalten, aber neben weiteren pharmazeutischen Wirkstoffen auch Salze, Puffer, Vitamine, Zuckerderivate, insbesondere Saccaride, Enzyme, Pflanzenextrakte und andere. Die Puffer und Zuckerderivate reduzieren mit Vorteil den Schmerz bei subkutaner Applikation und Enzyme wie beispielsweise Hyaluronidase erhöhen die Wirksamkeit. Die Herstellung der oben aufgeführten pharmazeutischen Zubereitungen erfolgt in üblicher Weise nach bekannten Methoden, zum Beispiel durch Mischen des oder der Wirkstoffe mit dem oder den Trägerstoffen.

Die genannten Zubereitungen können bei Mensch und Tier entweder oral, rektal, parenteral (intravenös, intra-muskulär, subkutan), intracisternal, intravaginal, intraperitoneal, lokal (Puder, Salbe, Tropfen) und zur Therapie der unten genannten Krankheiten angewendet werden. Als geeignete Zubereitung kommen Injektionslösungen, Lösungen und Suspensionen für die orale Therapie, Gele, Aufgussformulierungen, Emulsionen, Salben oder Tropfen in Frage. Zur lokalen Therapie können ophtalmologische und dermatologische Formulierungen, Silber- und andere Salze, Ohrentropfen, Augensalben, Puder oder Lösungen verwendet werden. Bei Tieren kann die Aufnahme auch über das Futter oder Trinkwasser in geeigneten Formulierungen erfolgen. Ferner können die Arzneimittel in andere Trägermaterialien wie zum Beispiel Kunststoffe - Kunststoffketten zur lokalen Therapie -, Kollagen oder Knochenzement eingearbeitet werden.

In einer weiteren bevorzugten Ausführungsform der Erfindung sind die Verbindungen in einer Konzentration von 0,1 bis 99,5, bevorzugt von 0,5 bis 95, besonders bevorzugt von 20 bis 80 Gew.-% in einer pharmazeutischen Zubereitung eingebracht. Das heißt, die Verbindungen sind in den oben aufgeführten pharmazeutischen Zubereitungen, zum Beispiel Tabletten, Pillen, Granulaten und anderen, vorzugsweise in einer Konzentration von 0,1 bis 99,5 Gew.-% der Gesamtmischung vorhanden. Die Wirkstoffmenge, das heißt die Menge der erfindungsgemäßen Verbindungen, die mit den Trägermaterialien kombiniert wird, um eine einzige Dosierungsform zu erzeugen, wird von dem Fachmann in Abhängigkeit von dem zu behandelnden Patienten und der besonderen Verabreichungsart variieren können. Nach Besserung des Zustandes des Patienten kann der Anteil der wirksamen Verbindung in der Zubereitung so geändert werden, dass eine Erhaltungsdosis vorliegt, die die Krankheit zum Stillstand bringt. Anschließend kann die Dosis oder Frequenz der Verabreichung oder beides als Funktion der Symptome auf eine Höhe verringert werden, bei der der verbesserte Zustand beibehalten wird. Wenn die Symptome auf das gewünschte Niveau gelindert worden sind, sollte die Behandlung aufhören. Patienten können jedoch eine Behandlung mit Unterbrechung auf Langzeitbasis nach beliebigem Wiederauftreten von Erkrankungssymptomen benötigen. Demgemäß ist der Anteil der Verbindungen, das heißt ihre Konzentration, in der Gesamtmischung der pharmazeutischen Zubereitung ebenso wie ihre Zusammensetzung oder Kombination variabel und kann vom Fachmann aufgrund seines Fachwissens modifiziert und angepasst werden.

Dem Fachmann ist bekannt, dass die erfindungsgemäßen Verbindungen mit einem Organismus, bevorzugt einem Menschen oder einem Tier, auf verschiedenen Wegen in Kontakt gebracht werden können. Weiterhin ist dem Fachmann bekannt, dass insbesondere die pharmazeutischen Mittel in verschiedenen Dosierungen appliziert werden können. Die Applikation sollte hierbei so erfolgen, dass die Erkrankung möglichst effektiv bekämpft wird bzw. der Ausbruch einer Krankheit in einer prophylaktischen Gabe verhindert wird. Die Konzentration und die Art der Applikation können vom Fachmann durch Routineversuche eruiert werden. Bevorzugte Applikationen der erfindungsgemä-ßen Verbindungen sind die orale Applikation in Form von Pulver, Tabletten, Saft, Tropfen, Kapseln oder ähnlichem, die rektale Applikation in Form von Zäpfchen, Lösungen und ähnlichem, parenteral in Form von Injektionen, Infusionen und Lösungen sowie lokal in Form von Salben, Pflastern, Umschlägen, Spülungen und ähnlichem. Bevorzugt erfolgt das In-Kontakt-Bringen der erfindungsgemäßen Verbindungen prophylaktisch oder therapeutisch.

Die Eignung der gewählten Applikationsformen wie auch der Dosis, des Applikationsschemas, der Adjuvantswahl und dergleichen kann beispielsweise durch Entnahme von Serum-Alliquoten aus dem Patienten, das heißt dem Mensch oder dem Tier, und dem Testen auf das Vorhandensein von Krankheitsindikatoren im Verlauf des Behandlungsprotokolls bestimmt werden. Alternativ und begleitend hierzu kann der Zustand der Nieren, der Leber o. a., aber auch die Menge von T-Zellen oder anderen Zellen des Immunsystems, auf herkömmliche Weise begleitend bestimmt werden, um einen Gesamtüberblick über die immunologische Konstitution des Patienten und insbesondere die Konstitution von stoffwechselwichtigen Organen, zu erhalten. Zusätzlich kann der klinische Zustand des Patienten auf die gewünschte Wirkung hin beobachtet werden. Wenn eine unzureichende therapeutische Effektivität erzielt wird, kann der Patient mit erfindungsgemäßen Mitteln ggf. mit anderen bekannten Medikamenten modifiziert weiterbehandelt werden, von denen eine Verbesserung der Gesamtkonstitution erwartet werden kann. Selbstverständlich ist es auch möglich, die Träger oder Vehikeln des pharmazeutischen Mittels zu modifizieren oder den Verabreichungsweg zu variieren.

Neben der oralen Aufnahme kann es dann zum Beispiel vorgesehen sein, dass Injektionen beispielsweise intramuskulär oder subkutan oder in die Blutgefäße ein weiterer bevorzugter Weg für die therapeutische Verabreichung der erfindungsgemäßen Verbindungen sind. Zeitgleich kann auch die Zufuhr über Katheter oder chirurgische Schläuche angewendet werden; beispielsweise über Katheter, die direkt zu bestimmten Organen wie den Nieren, der Leber, der Milz, dem Darm, der Lunge etc. führen.

Die erfindungsgemäßen Verbindungen können in einer bevorzugten Ausführungsform in einer Gesamtmenge von bevorzugt 0,05 bis 500mg/kg Körpergewicht je 24 Stunden eingesetzt werden, bevorzugt von 5 bis 100mg/kg Körpergewicht. Hierbei handelt es sich vorteilhafterweise um eine therapeutische Menge, die verwendet wird, um die Symptome einer Störung oder responsiven, pathologisch physiologischen Kondition zu verhindern oder zu verbessern.

Selbstverständlich wird die Dosis vom Alter, der Gesundheit und dem Gewicht des Empfängers, dem Grad der Krankheit, der Art einer notwendigen gleichzeitigen Behandlung, der Häufigkeit der Behandlung und der Art der gewünschten Wirkungen und der Nebenwirkungen abhängen. Die tägliche Dosis von 0,05 bis 500mg/kg Körpergewicht kann einmalig oder mehrfach angewendet werden, um die gewünschten Ergebnisse zu erhalten. Typischerweise werden insbesondere pharmazeutischen Mittel zur etwa 1-bis 10-maligen Verabreichung pro Tag oder alternativ oder zusätzlich als kontinuierliche Infusion verwendet. Solche Verabreichungen können als chronische oder akute Therapie angewendet werden. Die Wirkstoffmengen, die mit den Trägermaterialien kombiniert werden, um eine einzelne Dosierungsform zu erzeugen, können in Abhängigkeit von dem zu behandelnden Wirt und der besonderen Verabreichungsart selbstverständlich variieren. Bevorzugt ist es, die Tagesdosis auf 2 bis 5 Applikationen zu verteilen, wobei bei jeder Applikation zum Beispiel 1 bis 2 Tabletten mit einem Wirkstoffgehalt von 0,05 bis 500mg/kg Körpergewicht verabreicht werden. Selbstverständlich ist es möglich, den Wirkstoffgehalt auch höher zu wählen, beispielsweise bis zu einer Konzentration bis 5000mg/kg. Die Tabletten können auch retardiert sein, wodurch sich die Anzahl der Applikationen pro Tag auf 1 bis 3 vermindert. Der Wirkstoffgehalt der retardierten Tabletten kann 3 bis 3000mg betragen. Wenn der Wirkstoff - wie ausgeführt - durch eine Injektion verabreicht wird, ist es bevorzugt, 1- bis 10-mal pro Tag beziehungsweise durch Dauerinfusion den Wirt mit den erfindungsgemäßen Verbindungen in Kontakt zu bringen, wobei Mengen von 1 bis 4000mg pro Tag bevorzugt sind. Die bevorzugten Gesamtmengen pro Tag haben sich in der Humanmedizin und in der Veterinärmedizin als vorteilhaft erwiesen. Es kann erforderlich sein, von den genannten Dosierungen abzuweichen und zwar in Abhängigkeit von der Art und dem Körpergewicht des zu behandelnden Wirts, der Art und der Schwere der Erkrankung, der Art der Zubereitung der Applikation des Arzneimittels sowie dem Zeitraum bzw. dem Intervall, innerhalb welchem die Verabreichung erfolgt. So kann es in einigen Fällen bevorzugt sein, den Organismus mit weniger als den genannten Mengen in Kontakt zu bringen, während in anderen Fällen die angegebene Wirkstoffmenge überschritten werden muss. Die Festlegung der jeweils erforderlichen optimalen Dosierungen und der Applikationsart der Wirkstoffe kann durch den Fachmann aufgrund seines Fachwissens erfolgen.

In einer weiteren besonders bevorzugten Ausführungsform der Erfindung wird das pharmazeutische Mittel in einer Einzelgabe von 1 bis 100, insbesondere von 2 bis 50mg/kg Körpergewicht eingesetzt. Wie auch die Gesamtmenge pro Tag (siehe oben) kann auch die Menge der Einzelgabe pro Applikation von dem Fachmann aufgrund seines Fachwissens variiert werden. Die erfindungsgemäß verwendeten Verbindungen können in den genannten Einzelkonzentrationen und Zubereitungen zusammen mit dem Futter bzw. mit Futterzubereitungen oder mit dem Trinkwasser auch in der Veterinärmedizin gegeben werden. Eine Einzeldosis enthält vorzugsweise die Menge Wirkstoff, die bei einer Applikation verabreicht wird, und die gewöhnlich einer ganzen, einer halben Tagesdosis oder einem Drittel oder einem Viertel einer Tagesdosis entspricht. Die Dosierungseinheiten können demgemäß bevorzugt 1, 2, 3 oder 4 oder mehrere Einzeldosen oder 0,5, 0,3 oder 0,25 einer Einzeldosis enthalten. Bevorzugt wird die Tagesdosis der erfindungsgemäßen Verbindungen auf 2 bis 10 Applikationen verteilt, bevorzugt auf 2 bis 7, besonders bevorzugt auf 3 bis 5 Applikationen. Selbstverständlich ist auch eine Dauerinfusion der erfindungsgemäßen Mittel möglich.

In einer besonders bevorzugten Ausführungsform der Erfindung werden bei jeder oralen Applikation der erfindungsgemäßen Verbindungen 1 bis 2 Tabletten gegeben. Die erfindungsgemäßen Tabletten können mit dem Fachmann bekannten Überzügen und Hüllen versehen sein und auch so zusammengesetzt werden, dass sie den oder die Wirkstoffe nur bei bevorzugten, in einem bestimmten Teil des Wirts freigeben.

Bevorzugt ist es in einer weiteren Ausführungsform der Erfindung, dass die einzelnen Bestandteile der Verbindungen gegebenenfalls miteinander assoziiert oder mit einem Träger verbunden in Liposomen eingeschlossen sind, wobei der Einschluss in Liposomen im Sinne der Erfindung nicht zwingend bedeuten muss, dass die Verbindungen im Inneren der Liposomen vorliegen. Ein Einschluss im Sinne der Erfindung kann auch bedeuten, dass die Verbindungen mit der Membran der Liposomen assoziiert sind, beispielsweise so, dass diese auf der äußeren Membran verankert sind. Eine solche Darstellung der erfindungsgemäßen Verbindungen in oder auf den Liposomen ist vorteilhaft, wenn der Fachmann die Liposomen so auswählt, dass sie eine immunstimulierende Wirkung haben. Dem Fachmann sind aus der DE 198 51 2 82 verschiedene Möglichkeiten bekannt, die immunstimulierende Wirkung von Liposomen zu modifizieren. Bei den Lipiden kann es sich um einfache Lipide handeln, wie beispielsweise Ester und Amide oder um komplexe Lipide wie zum Beispiel um Glycolipide wie Cerebroside oder Ganglioside, um Sphingolipide oder um Phospholipide.

Bevorzugte Erkrankungen, die mit einer Defizienz des zellulären Immunsystems verbunden sind, die erfindungsgemäß behandelt werden können, sind im Sinne der Erfindung weiterhin: AIDS, Akne, Albuminurie (Proteinurie), Alkoholentzugssyndrom, Allergien, Alopezie (Haarausfall), ALS (Amyotrophe Lateralsklerose), Alzheimer Erkrankung, AMD (Altersbedingte Makuladegeneration), Anämie, Angststörungen, Anthrax (Milzbrand), Aortensklerose, arterielle Verschlusskrankheit, Arterienverkalkung, Arterienverschluss, Arteriitis temporalis, Arteriosklerose, Arteriovenöse Fisteln, Arthritis, Arthrose, Asthma, Ateminsuffizienz, Autoimmunerkrankung, AV-Block, Azidose, Bandscheibenvorfall, Bauchfellentzündung, Bauchspeicheldrüsenkrebs, Becker Muskeldystrophie, Benigne Prostata Hyperplasie (BPH), Blasenkarzinom, Bluterkrankheit (Hämophilie), Bronchialkarzinom, Brustkrebs, BSE, Budd-Chiari-Syndrom, Bulimia nervosa, Bursitis (Schleimbeutelentzündung), Byler-Syndrom, Bypass, Chlamydien-Infektion, Chronische Schmerzen, Cirrhose, Commotio cerebri (Gehirnerschütterung), Creutzfeld-Jakob-Erkrankung, Darmkarzinom, Darmkrebs, Darmtuberkulose, Depression, Diabetes insipidus, Diabetes mellitus, Diabetes mellitus juvenilis, Diabetische Retinopathie, Duchenne-Muskeldystrophie, Duodenalkarzinom, Dystrophia musculorum progressiva, Dystrophie, Ebola, Ekzem, Erektile Dysfunktion, Fettsucht, Fibröse, Gebärmutterhalskrebs, Gebärmutterkrebs, Gehirnblutung, Gehirnentzündung, Haarausfall, Halbseitenlähmung, Hämolytische Anämie, Hämophilie, Haustierallergie (Tierhaarallergie), Hautkrebs, Herpes zoster, Herzinfarkt, Herzinsuffizienz, Herzklappenentzündung, Hirnmetastase, Hirnschlag, Hirntumor, Hodenkrebs, Ischämie, Kahler-Krankheit (Plasmozytom), Kinderlähmung (Poliomyelitis), Knochenschwund, Kontaktekzem, Lähmung, Leberzirrhose, Leukämie, Lungenfibrose, Lungenkrebs, Lungenödem, Lymphdrüsenkrebs (Morbus Hodgkin), Lymphogranulomatose, Lymphom, Lyssa, Magenkarzinom, Mammakarzinom, Meningitis, Milzbrand, Mukoviszidose (zystrische Fibröse), Multiple Sklerose (MS), Myokardinfarkt, Neurodermitis, Neurofibro-matose, Neuronale Tumoren, Nierenkrebs (Nierenzellkarzinom), Osteoporose, Pankreaskarzinom, Pneumonie, Polyneuropathien, Potenzstörungen, Progressive Systemische Sklerose (PSS), Prostatakrebs, Quaddelauschlag (Urtikaria), Querschnitts-syndrom, traumatisches, Rektumkarzinom, Rippenfellentzündung, Schädel-Hirn-Trauma, Scheidenkrebs (Vaginalkarzinom), Sinusitis, Speiseröhrenkrebs, Tremor, Tuberkulose, Tumorschmerz, Vaginalkarzinom, Verbrennung, Verbrühung, Vergiftungen, Virale Meningitis, Wechseljahre, Weichteilsarkom, Weichteiltumor, Zerebrale Durchblutungsstörungen und/oder ZNS-Tumore, insbesondere ist die bevorzugte Erkrankung Bluthochdruck und mit Bluthochdruck assoziierte Erkrankungen.

Im Folgenden soll die Erfindung anhand von Beispielen näher erläutert werden, ohne auf diese Beispiele beschränkt zu sein.

### Ergebnisse:

Die Anwendung des neuen Produkts wurde bei andauernder Therapie angefangen und zwar aus zwei Gründen:
1. Die aprupte Abstellung der Therapie ruft das Reboundsyndrom hervor, insbesondere bei Anwendung der Alpha- und Betablocker sowie des Inhibitors ACE-Angetensin Converting Enzym.
2. Die natürlichen Produkte wirken langsamer. Daher sollte die vorhandene Therapie einige Zeit beibehalten werden.

### PATIENTEN

Bei 25 Patienten, die an einer unregulierten Hypertension leiden, bei kombinierter Therapie Betablocker plus Diuretika (185/109), wurde 1 Kapsel Nanopressan zusammen mit der konventionellen Therapie angewendet. Nach einem Monat sank der Blutdruck auf 145/92, nach zwei Monaten mit 2 Kapseln täglich auf 130/78. (Siehe Tabellen I, II, III)

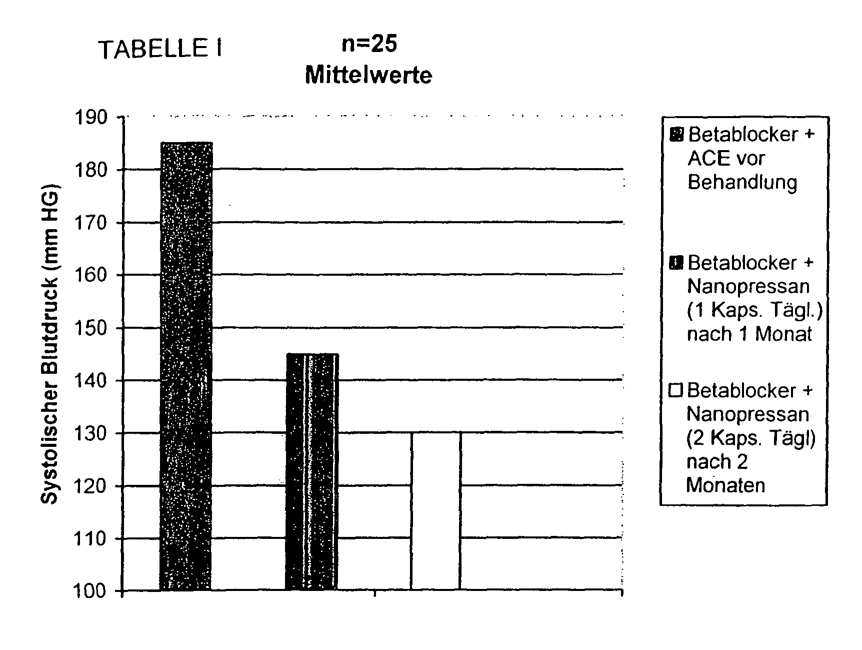

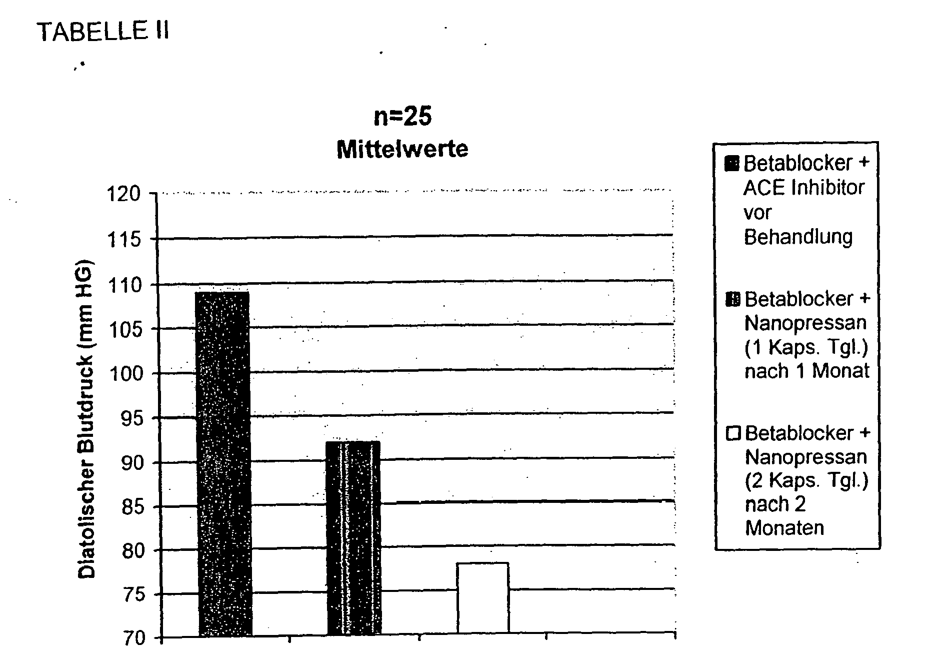

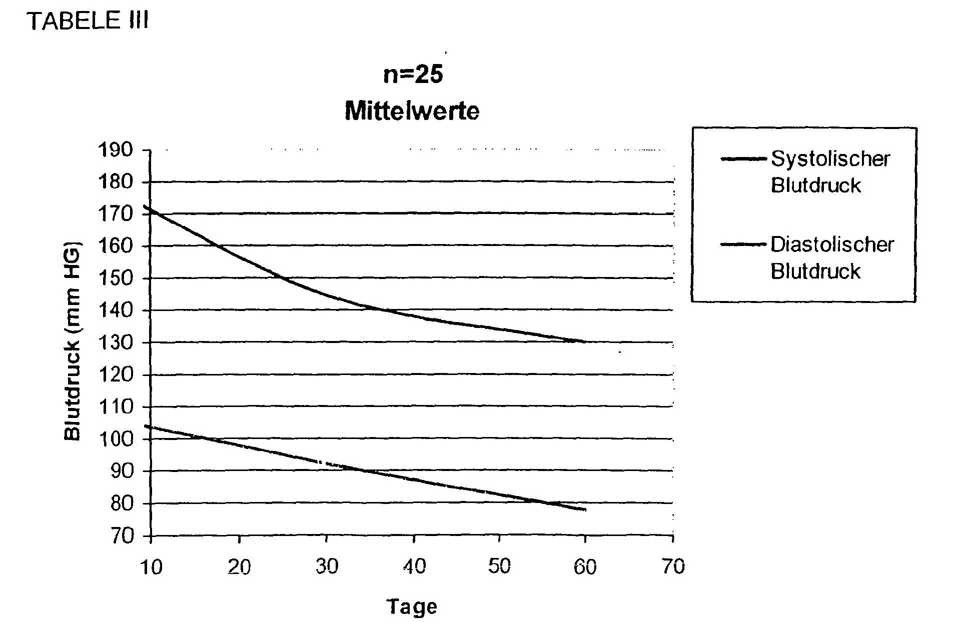

Bei regulierter Hypertension ersten Grades wurde bei 20 Patienten nach einem Monat die klassische Therapie abgestellt, sodass die lediglich Nanopressan zu sich nahmen. (Tabellen IV, V, VI)

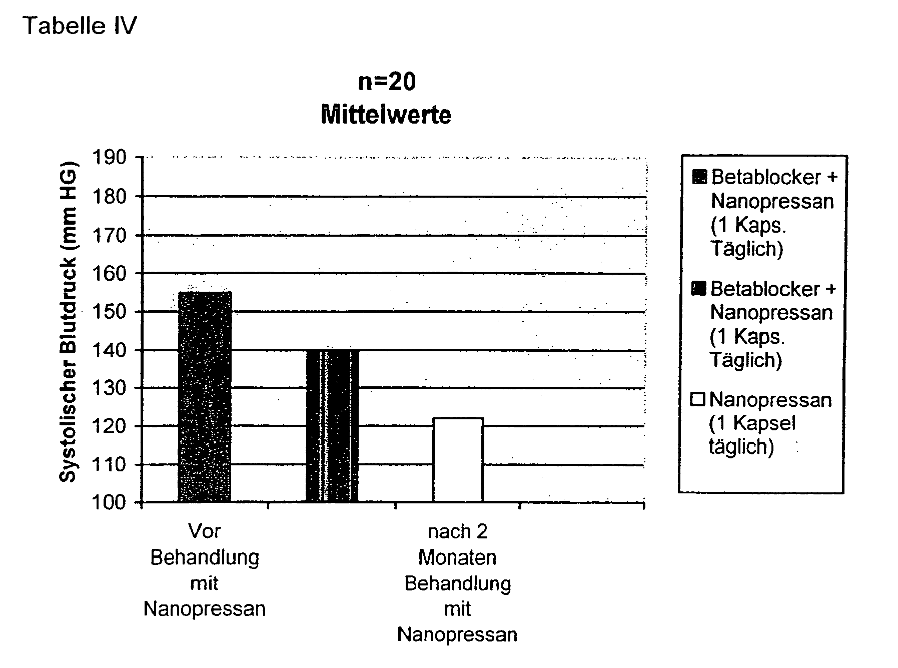

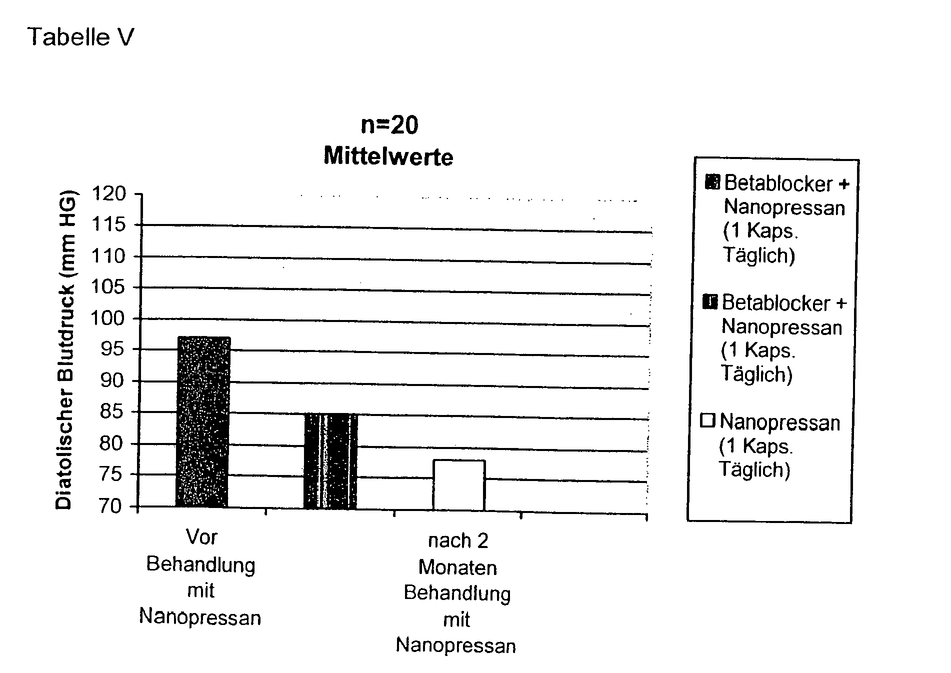

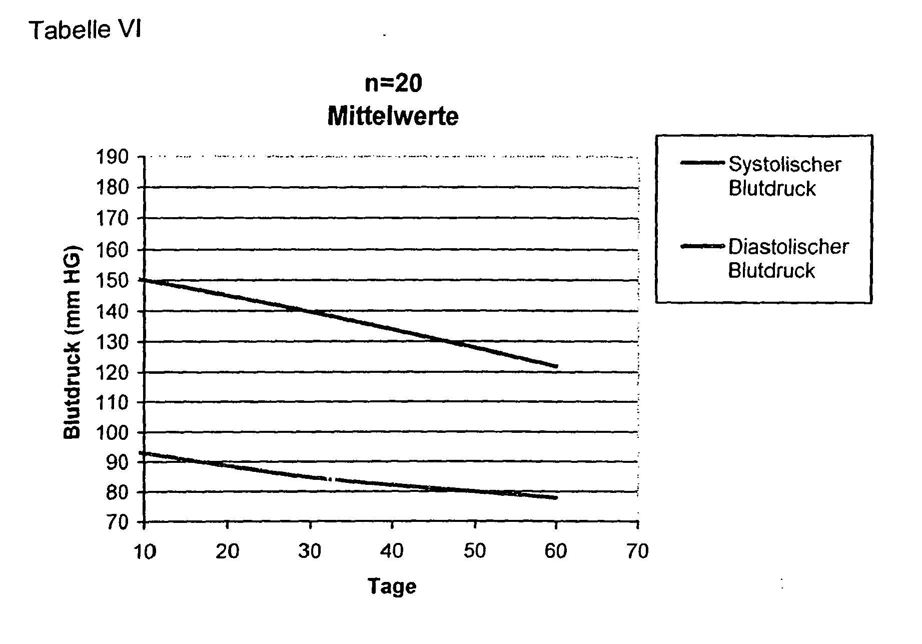

### Schlussfolgerung:

Nanopressan - Lebensmittel mit Wirkung auf den Blutdruck - enthält Substanzen, die
- auf den Volumenanteil des zirkulierenden Raums wirken;
- auf den vasokonstriktorischen Effekt(Verengung der Blutadern) wirken;
- auf den dilatatorischen Effekt (Erweiterung der Blutadern) wirken;
- den Herzrhythmus verlangsamen;
- einen diuretischen Effekt haben bzw. eine Verringerung des zirkulierenden Volumens hervorrufen;
- Schäden der Endotele verhindern, indem sie die Elastizität der Blutadern steigern;
- ACE sowie die Entstehung von ANG II inhibieren;
- einen Effekt auf die K/Na-AT-pase haben.

Diese Substanzen wirken nicht nur auf den erhöhten Blutdruck, sondern sie verhindern auch die Entstehung von Veränderungen an Blutadern, die einen erhöhten Blutdruck hervorrufen, insbesondere im höheren Alter oder beim immer häufiger auftretenden Stoffwechselsyndrom (zu 60% unter der älteren Population).

Die präventive Einnahme wird zweifellos eine wichtige Rolle spielen, da dies die einzige Möglichkeit ist, damit es nicht zur dauerhaften Verringerung der Elastizität der Blutadernwände kommt .

Unsere Erkenntnisse bezüglich der Krankheitsprävention werden jeden Tag wichtiger, da sie die einzig wirksame und wesentlich preiswertere Methode darstellt, als wenn dauerhafte Schäden entstehen.

Bis jetzt ist kein Medikament bekannt, welches in einer Kapsel Substanzen enthält, die Einfluss haben auf alle Mechanismen der antihypertensiven Wirkung.

An dieser Stelle soll angemerkt werden, dass es sich hier um natürliche Substanzen handelt, die weder Nebeneffekte noch toxische Folgen für Leber, Nieren und andere Organe haben, was vor allem bei längerfristiger Anwendung von Bedeutung sein wird.

Am bekanntesten ist die nachgewiesene Tatsache, dass Nanopressan den systolischen und den diastolischen Blutdruck gleichmäßig senkt (siehe Graphik). Daraus können wir schlussfolgern, dass dieses Produkt das Medikament der Zukunft für die Regulierung der Hypertension und die Erhaltung des für die Gesundheit des Menschen außerordentlich wichtigen Blutadersystems ist.

## Patentansprüche

1. Pharmazeutisches Mittel umfassend TMAZ und einen Pflanzenextrakt ausgewählt aus der Gruppe umfassend Bärlauchkraut, Mistelkraut, Silberlindenblüten, Weißdornblüten und/oder Gingkoblätter.

2. Pharmazeutisches Mittel umfassend 50 - 90 Gew.-% TMAZ, 1 - 30 Gew.-% Bärlauchkraut, 1 - 10 Gew.-% Mistelkraut, 1 - 10 Gew.-% Silberlindenblüten, 1 - 10 Gew.-% Weißdornblüten und/oder 1 - 10 Gew.-% Gingkoblätter und ggf. einen pharmazeutisch verträglichen Träger, Adjuvanz und/oder Vehikel enthält.

3. Mittel nach Anspruch 1 oder 2,
**dadurch gekennzeichnet, dass**
es 60 - 80 Gew.-% TMAZ, 7 - 13 Gew.-% Bärlauchkraut, 3 - 7 Gew.-% Mistelkraut, 3 - 7 Gew.-% Silberlindenblüten, 3 - 7 Gew.-% Weißdornblüten und/oder 3 - 7 Gew.-% Gingkoblätter umfasst.

4. Mittel nach einem der vorhergehenden Ansprüche,
**dadurch gekennzeichnet, dass**
es 70 Gew.-% TMAZ, 10 Gew.-% Bärlauchkraut, 5 Gew.-% Mistelkraut, 5 Gew.-% Silberlindenblüten, 5 Gew.-% Weißdornblüten und/oder 5 Gew.-% Gingkoblätter umfasst.

5. Mittel nach einem der vorhergehenden Ansprüche,
**dadurch gekennzeichnet, dass**
der Träger ausgewählt ist aus der Gruppe umfassend Füllmittel, Streckmittel, Bindemittel, Feuchthaltemittel, Sprengmittel, Lösungsverzögerer, Resorptionsbeschleuniger, Netzmittel, Adsorptionsmittel und/oder Gleitmittel

6. Mittel nach einem der vorhergehenden Ansprüche,
**dadurch gekennzeichnet, dass**
die Vehikel Liposomen sind.

7. Mittel nach einem der vorhergehenden Ansprüche,
**dadurch gekennzeichnet, dass**
es ein Gel, ein Puder, ein Pulver, eine Infusionslösung, eine Tablette, eine Retard-Tablette, ein Pre-Mix, ein Prodrug, eine Emulsion, eine Aufgussformulierung, ein Tropfen, ein Konzentrat, ein Granulat, ein Sirup, Pellet, ein Boli, eine Kapsel, ein Aerosol, ein Spray und/oder ein Inhalat ist.

8. Kombinationsmittel umfassend ein Mittel gemäß einem der Ansprüche 1 - 6 und ein Mittel ausgewählt aus der Gruppe umfassend ACE-Hemmer, Angiotensin-II-Rezeptorantagonisten, Diuretika, Betablocker, Alphablocker und/oder Kalziumblocker.

9. Verwendung eines Mittels gemäß einem der Ansprüche 1 - 8 zur Prophylaxe, Therapie, Verlaufskontrolle und/oder Nachbehandlung von Erkrankungen, die mit Bluthochdruck im Zusammenhang stehen.

10. Verwendung nach dem vorhergehenden Anspruch,
**dadurch gekennzeichnet, dass**
die Erkrankungen ausgewählt sind aus der Gruppe umfassend Kopfschmerzen, Übelkeit, Erbrechen, Taubheitsgefühl, Nasenbluten, schwere Atemnot und/oder Angina pectoris.

11. Verwendung nach Anspruch 10,
**dadurch gekennzeichnet, dass**
der Bluthochdruck ausgewählt ist aus der Gruppe umfassend arterielle Hypertonie, pulmonare Hypertonie, venöse Stauung und/oder portale Hypertonie.

12. Verwendung nach dem vorhergehenden Anspruch,
**dadurch gekennzeichnet, dass**
ein pharmazeutisches Mittel gemäß einem der Ansprüche 1 - 7 in einer Konzentration von 0,1 - 99,5 Gew.-%, bevorzugt von 0,5 - 95,0 Gew.-%, besonders bevorzugt von 20 - 80 Gew.-% in einer Zubereitung vorliegen.

13. Verwendung nach dem vorhergehenden Anspruch,
**dadurch gekennzeichnet, dass**
die Zubereitung oral, subkutan, intravenös, intramuskulär, intraperitoinal und/oder thopisch eingesetzt wird.

14. Verwendung nach einem der vorhergehenden Ansprüche,
**dadurch gekennzeichnet, dass**
ein pharmazeutisches Mittel gemäß einem der Ansprüche 1 - 8 in Gesamtmengen von 0,05 - 500 mg pro kg Körpergewicht, bevorzugt von 5 - 100 mg pro kg Körpergewicht je 24 Stunden eingesetzt wird.

15. Kit umfassend mindestens ein pharmazeutisches Mittel gemäß Anspruch 1 - 8, ggf. mit einer Information zum Kombinieren der Inhalte des Kits.

16. Verwendung des Kits nach dem vorhergehenden Anspruch zur Prophylaxe oder Therapie von Bluthochdruckerkrankungen.

17. Verfahren zur Behandlung des Bluthochdrucks,
**dadurch gekennzeichnet, dass**
ein Mittel gemäß einem der Ansprüche 1 - 8 mit einem Patienten in Kontakt gebracht wird.

18. Verfahren nach dem vorhergehenden Anspruch,
**dadurch gekennzeichnet, dass**
die Bestandteile des Kombinationsmittels nach Anspruch 8 zeitgleich oder sequentiell mit dem Patienten in Kontakt gebracht werden.

19. Verfahren nach dem vorhergehenden Anspruch,
**dadurch gekennzeichnet, dass**
das in Kontakt Bringen bevorzugt oral erfolgt.
